# EUROPEAN PATENT APPLICATION

(11) **EP 4 365 908 A1**
(43) Date of publication of application: **08.05.2024**
(21) Application number: 22205470.2
(22) Date of filing: 04.11.2022
(51) Int. Cl.: G16H 30/20, G06N 3/0455, G06T 9/00, H04N 19/00

(54) **COMPRESSION OF MEASUREMENT DATA FROM MEDICAL IMAGING SYSTEM**

(71) Applicant: Koninklijke Philips N.V., 5656 AG Eindhoven (NL)
(72) Inventor: GOEDICKE, Andreas Georg, Eindhoven (NL); PEZZOTTI, Nicola, Eindhoven (NL); BERGNER, Frank, 5656AG Eindhoven (NL); GRASS, Michael, 5656AG Eindhoven (NL); NETSCH, Thomas, Eindhoven (NL); CABALLERO, Jose Maria, Eindhoven (NL); SCHNELLBÄCHER, Nikolas David, Eindhoven (NL)
(74) Representative: Philips Intellectual Property & Standards

(57) **Abstract**

Disclosed herein is a medical system (100) comprising a local memory (124) storing local machine executable instructions (130) and a data compression module (140). the data compression module comprises an encoder portion of an autoencoder. The medical system further comprises a medical imaging system (108) configured to acquire measurement data (134) descriptive of a subject (110). The medical system further comprises a local computational system (118). Execution of the local machine executable instructions causes the local computational system to: control (200) the medical imaging system to acquire the measurement data; construct (202) compressed measurement data (138) by compressing the measurement data using the data compression module, wherein the compressed data is derived from a latent space vector of the autoencoder; and transmit (204) the compressed data to a remote computational system (150) via a network connection (106).

## Description

### FIELD OF THE INVENTION

The invention relates to medical imaging, in particular to the remote reconstruction of medical images.

### BACKGROUND OF THE INVENTION

Various tomographic medical imaging techniques such as Magnetic Resonance Imaging (MRI), Computed Tomography, Positron Emission Tomography, and Single Photon Emission Tomography enable detailed visualization of anatomical structure of a subject. It is possible to acquire medical imaging data locally and then use a remote computing system to perform potentially computationally intensive reconstruction of a medical image from the measurement data.

United States patent application publication US2021027169A1 discloses a system and method for training a parametric machine learning system, include compressing a first data; storing the compressed first data; reconstructing a first selected amount of the stored compressed first data; providing a machine learning system; and training the machine learning system with the reconstructed first data and optionally raw data.

### SUMMARY OF THE INVENTION

The invention provides for a medical system, a computer program, and a method in the independent claims. Embodiments are given in the dependent claims.

A difficulty with using remote computational systems, such as a server or cloud-based systems, to reconstruct medical imaging data is that large quantities of data may need to be transmitted to perform the reconstruction. Embodiments may provide for an improved means of compressing measurement data in a transmitter portion by inputting the measurement data into encoder portion of an autoencoder to produce a latent space vector which is used to construct compressed measurement data. A receiver portion at the remote location then decodes the compressed measurement data using the decoder portion of the autoencoder. A benefit of using the encoder portion and decoder portion of an autoencoder may be that the compression can be very well tailored to the particular measurement data being compressed. Better compression ratios can for example be obtained than traditional compression algorithms such as a zip compression.

In one aspect the invention provides for a medical system that comprises a local memory storing machine-executable instructions and a data compression module. The data compression module comprises an encoder portion of an autoencoder. An autoencoder, as used herein, encompasses a neural network that receives data as input and then reproduces the data at the output. Typically, the autoencoder comprises an encoder portion and a decoder portion. The encoder portion produces a so-called latent space vector, which is then input into the decoder portion. The autoencoder may be used as a data compression module because the latent space vector comprises less data than the original data that was input into it.

The medical system further comprises a medical imaging system that is configured to acquire measurement data that is descriptive of a subject. The medical imaging system may for example be various types of tomographic medical imaging such as computed tomography or magnetic resonance imaging. The medical imaging system may also encompass other types of medical imaging such as ultrasound systems or various types of X-ray or fluoroscopy systems. The measurement data as used herein encompasses the raw data measured by the medical imaging system. In different examples this may have different meanings. For example, in magnetic resonance imaging the frequency signals are sampled and this is so called k-space data, which may then be Fourier transformed into an image. In one example the measurement data are the k-space samples directly. However, various magnetic resonance imaging techniques such as parallel imaging may acquire k-space data from multiple coils, which is then constructed into individual coil images. The individual coil images may be combined with a variety of techniques into a final medical image. In some examples, the measurement data may refer to the k-space data and in other examples it may refer to the uncombined coil images. Likewise, in computed tomography the X-ray data may be acquired in different techniques. For example, various radiation profile measurements may be acquired. These individually acquired radiation profiles may be transmitted as the measurement data.

The medical system further comprises a local computational system. The term 'local computational system' is to refer or provide a label for a particular computational system. A local computational system as used herein is a computational system. The term 'local' is simply to provide a differentiating term between different computational systems. Likewise, the term 'local memory' is a label to refer to a particular memory and the 'local machine-executable instructions', the term 'local' is a label to identify a particular set of machine-executable instructions. Execution of the local machine-executable instructions causes the local computational system to control the medical imaging system to acquire the measurement data. For example, there may be a set of commands such as a pulse sequence, as is used in magnetic resonance imaging, to acquire the measurement data according to a particular protocol.

Execution of the local machine-executable instructions further causes the computational system to construct compressed measurement data by compressing the measurement data using the data compression module. The compressed data is derived from a latent space vector of the autoencoder. This may mean different things in different examples. In one example the compressed data may simply be the latent space vector. In other examples the latent space vector may be further compressed by an algorithmic or traditional compression technique. In other examples the compressed data may comprise additional data that is associated with or describes the latent space vector or a compressed equivalent of it. Execution of the machine-executable instructions further causes the local computational system to transmit the compressed data to a remote computational system via a network connection.

This embodiment may be beneficial because the encoder portion of the autoencoder may be trained to compress particular measurement data. For example, if the measurement data is from a magnetic resonance imaging system or a computed tomography system the encoder portion may be trained with the type of data which is to be compressed. This may result in a compression which works better than algorithms designed for general data compression.

Various types of neural networks may be used for implementing the autoencoder. For example, a U-Net, a 2-scale Vector-Quantized Variational Autoencoder, and a variational autoencoder may be used.

The autoencoder may be trained by taking exemplary data from the medical imaging system that has been acquired as measurement data and using this as training data. Various groups of measurement data may be input into the autoencoder and compared to what is output. The difference between these may then be used, for example, with a deep learning algorithm to modify the autoencoder. Once the autoencoder has been trained the encoder portion can be used for the data compression module and the decoder portion of the autoencoder can be used as a data decompression module. As can be seen, the training of an autoencoder to function for data compression may be very straightforward. As the data input into the autoencoder is compared to the data that is output, there is not a need to label the data. Large amounts of data which has been previously acquired by the medical imaging system can be prepared and used as training data.

In another embodiment the local memory further stores a data decompression module. The data decompression module comprises a decoder portion of the autoencoder. In this embodiment, the local memory also stores the decoder portion of the autoencoder and uses this as a data decompression module. This may be advantageous in various embodiments because it may enable the comparison of approximated measurement data which is obtained by decompressing the compressed data to the original data. This makes it possible to provide an estimate of the error.

In another embodiment the medical system further comprises a remote memory storing remote machine-executable instructions and the data decompression module. The medical system further comprises a remote computational system. In this context the term 'remote' for remote memory, remote machine-executable instructions, and remote computational system are a label to identify a memory, machine-executable instructions and computational system that are at a different location than the local memory, the local machine-executable instructions and the local computational system. As such, the remote memory is a memory, the remote machine-executable instructions are machine-executable instructions, and the remote computational system is a computational system.

Execution of the remote machine-executable instructions causes the remote computational system to receive the compressed measurement data via the network connection. Execution of the remote machine-executable instructions further causes the remote computational system to obtain approximated measurement data by decompressing the compressed measurement data with the decompression module. Execution of the remote machine-executable instructions causes the remote computational system to reconstruct the medical image at least partially from the approximated measurement data. This embodiment may be beneficial because, as was mentioned before, the use of an autoencoder may provide for a means of providing compression that is tailored to a particular type of data. For example, measurement data from a particular type of medical imaging system. Another advantage is that autoencoders tend to provide an approximation of the original data such as an image or other data that was input into it. A benefit of approximating the original measurement data with the autoencoder is that it has a tendency to remove noise and may provide for a medical image that has been denoised. The denoising of the medical image by approximating its data with the autoencoder may provide for a superior denoising in comparison with other algorithms.

In another embodiment execution of the remote machine-executable instructions further causes the remote computational system to perform a noise filtering on the approximated measurement data before reconstruction into the medical image. This embodiment may be beneficial because it may provide for a means of applying a specific noise filtering algorithm to the approximated measurement data in a remote computational setting. Various noise filtering algorithms may be used such as conventional noise filtering or smoothing algorithms as well as using neural networks trained for noise filtering.

In another embodiment the local memory further comprises a secondary compression module. Execution of the local machine-executable instructions further causes the local computational system to obtain approximated measurement data by decompressing the compressed measurement data with the decompression module. Execution of the local machine-executable instructions further causes the local computational system to determine an error of the approximated measurement data by calculating a difference between the measurement data and the approximated measurement data. Execution of the machine-executable instructions further causes the local computational system to obtain compressed error data by compressing the error of the approximated measurement data with the secondary compression module. Execution of the machine-executable instructions further causes the local computational system to transmit the compressed measurement data with the compressed error data to the remote computational system via the network connection. This embodiment may be beneficial because it may provide for a means of reproducing the measurement data in a lossless fashion. When the compressed error data is decompressed it may be used to correct the approximated measurement data.

In another embodiment the secondary compression module is implemented as an algorithmic compression module. The algorithmic compression module may, for example, be a zip, tar or other conventional compression technique. As with the previously mentioned autoencoder, the second autoencoder may also be implemented as a U-Net, a 2-scale Vector-Quantized Variational Autoencoder, and a variational autoencoder.

The second autoencoder may be trained by taking previously acquired measurement data and using this to generate the error of the approximate measurement data as was described above. This may be used as training data for the second autoencoder.

In another embodiment the latent space vector is quantized. Quantizing the latent space vector may have the benefit that the compression becomes lossy in a predictable manner. The quantization of the latent space vector can be selected. An advantage of this type of lossy compression is that it has the effect of removing noise from the resulting approximated measurement data.

In another embodiment the data compression module is lossy. This may have the same benefit of having the latent space vector being quantized.

In another embodiment the autoencoder is a U-net. When using a U-net as an autoencoder it could be noted that the U-net has a more or less symmetric shape. The connection between the two halves of the U that form the U-net may be used to provide the latent space vector. For example, the U-net can be trained to reproduce the data that is input. The various connections at the lowest level of the U-net as well as the skipped connections of the U-net, are then used to provide the latent space of the U-net as an autoencoder.

In another embodiment the autoencoder is a two-scale vector-quantized variational autoencoder.

In another embodiment the autoencoder is a variational autoencoder.

In another embodiment execution of the local machine-executable instructions further causes the local computational system to perform a noise reduction on the measurement data before compression into the compressed data. This may be beneficial because it may have an effect of reducing the noise in the measurement data before it is compressed. This may for example be particularly useful when using a non-lossy compression scheme.

In another embodiment the medical imaging system is a computed tomography system.

In another embodiment the medical imaging system is a magnetic resonance imaging system.

In another embodiment the medical imaging system is a diagnostic ultrasound system.

In another embodiment the medical imaging system is a positron emission tomography system.

In another embodiment the medical imaging system is a single-photon emission tomography system.

In another aspect, the invention provides for a medical system that comprises a remote memory storing remote machine-executable instructions and a data decompression module. The data decompression module comprises a decoder portion of the autoencoder. The medical system further comprises a remote computational system. Execution of the remote machine-executable instructions causes the remote computational system to receive compressed measurement data via a network connection. The compressed measurement data is derived from a latent space vector of the autoencoder. Execution of the remote machine-executable instructions further causes the computational system to obtain approximated measurement data by decompressing the compressed measurement data with a decompression module. Execution of the remote machine-executable instructions further causes the remote computational system to reconstruct a medical image at least partially from the approximated measurement data.

In another embodiment the remote memory further comprises a secondary decompression module. Execution of the remote machine-executable instructions further causes the remote computational system to receive compressed error data. Execution of the machine-executable instructions further causes the remote computational system to obtain an error of the approximated measurement data by decompressing the compressed error data with the secondary decompression module. Execution of the remote machine-executable instructions further causes the remote computational system to calculate measurement data by correcting the approximated measurement data with the approximated measurement data. The medical image is reconstructed from the measurement data. This embodiment may be beneficial because it provides for a lossless means of very efficiently transferring the measurement data.

In another embodiment the step of transmitting the compressed data to the remote computational system also comprises sending metadata descriptive of the measurement data or the compressed data to the remote computational system. For example, the metadata could provide data descriptive of the imaging protocol or details used to acquire the measurement data. This may for example be used in selecting an algorithm for reconstruction of the medical image from the approximated measurement data.

In another aspect, the invention provides for a computer program comprising local machine-executable instructions for execution by a local computational system that is configured for controlling a medical system and a data compression module. The data compression module comprises an encoder portion of an autoencoder. The medical system comprises a medical imaging system that is configured to acquire measurement data descriptive of a subject.

Execution of the local machine-executable instructions causes the local computational system to control the medical imaging system to acquire the measurement data. Execution of the local machine-executable instructions further causes the local computational system to construct compressed measurement data by compressing the measurement data using the data compression module. The compressed data is derived from a latent space vector of the autoencoder. Execution of the local machine-executable instructions further causes the local computational system to transmit the compressed data to a remote computational system via a network connection.

In another aspect the invention provides for a method of operating a medical system. The medical system comprises a medical imaging system configured to acquire measurement data descriptive of a subject. The method comprises controlling the medical imaging system to acquire the measurement data using a local computational system. The method further comprises constructing compressed measurement data by compressing the measurement data using a data compression module using the local computational system. The data compression module comprises an encoder portion of an autoencoder. The compressed data is derived from a latent space vector of the autoencoder. The method further comprises transmitting the compressed data to a remote computational system via a network connection by the local computational system.

In another embodiment the method further comprises receiving compressed measurement data via a network connection via the remote computational system. The compressed measurement data is derived from a latent space vector of the autoencoder. The method further comprises obtaining approximated measurement data by decompressing the compressed measurement data with a decompression module using the remote computational system. The method further comprises reconstructing a medical image at least partially from the approximated measurement data using the remote computational system.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Measurement data is defined herein as being recorded measurements made by a medical imaging system descriptive of a subject. The medical imaging data may be reconstructed into a medical image. A medical image is defined herein as being the reconstructed two- or three- or four-dimensional visualization of anatomic data contained within the measurement data, also allowing for volumetric time-series data to be covered. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a first implementation and a second implementation of methods;
Fig. 4 illustrates a further implementation of a method;
Fig. 5 illustrates the compression and decompression of the measurement data into the approximated measurement data;
Fig. 6 illustrates the results of examples of the compression technique.
Fig. 7 shows a medical image reconstructed from a lossless compression of the measurement data and a medical image reconstructed from a lossy compression of the measurement data;
Fig. 8 illustrates a method of using a cascade of compression modules to construct the compressed measurement data and the compressed error data; and
Fig. 9 illustrates the lossless reconstruction of the measurement data after receiving the compressed measurement data and the compressed error data.

### DESCRIPTION OF EMBODIMENTS

Like numbered elements in these figures are either equivalent elements or perform the same function. Elements which have been discussed previously will not necessarily be discussed in later figures if the function is equivalent.

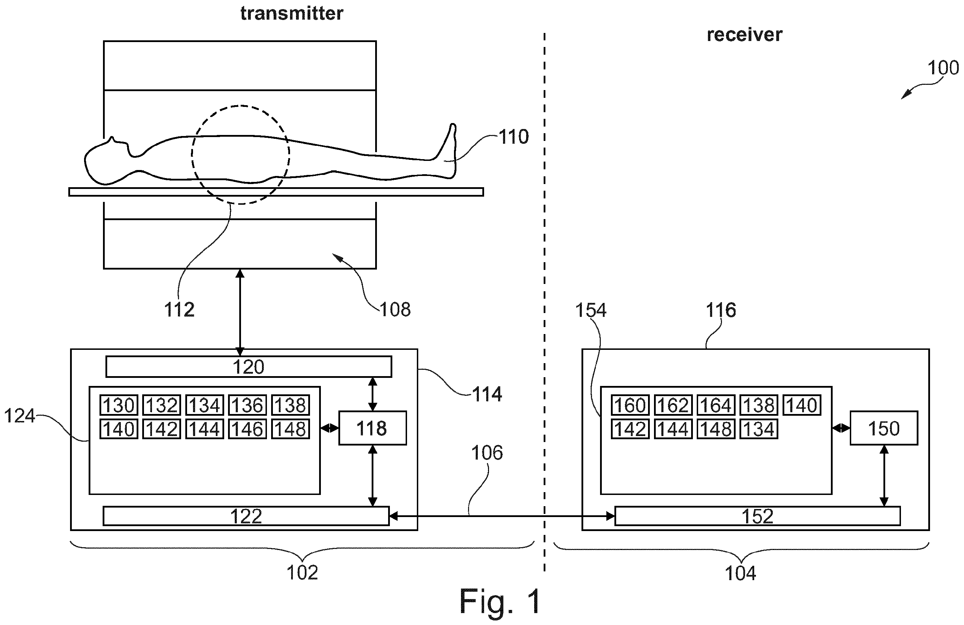

Fig. 1 illustrates an example of a medical system 100. The medical system 100 is divided into a transmitter portion 102 and a receiver portion 104 that communicate via a network connection 106. The transmitter portion 102 is shown as comprising a medical imaging system 108 and a local computer system 114. The receiver portion is shown as comprising a remote computer system 116. The remote computer system 116 could for example be a cloud-based or remote system that is provided for reconstructing various medical images.

The medical imaging system 108 is intended to represent various types of medical imaging systems and is shown as having a portion of a subject 110 within an imaging zone 112. The imaging zone 112 is where the measurement data 134 is acquired and provides data which is descriptive of anatomical structures of the subject 110. The medical imaging system could, for example be: a computed tomography system, a magnetic resonance imaging system, a diagnostic ultrasound system, a positron emission tomography system, and a single photon emission tomography system.

The local computer system 114 is shown as comprising a local computational system 118. The local computational system 118 may represent one or many local processing or computational cores. The local computational system 118 is shown as being in connection or in communication with a hardware interface 120, a local network interface 122 and a local memory 124. The hardware interface 120 enables the computational system 118 to communicate with and control the medical imaging system 108. The network interface 122 enables the computational system 118 to form one end of the network connection 106. The local memory 124 is intended to represent various types of memories which may be in communication with the computational system 118. In one example, the local memory is a non-transitory storage medium.

In some examples the local hardware interface 120 is identical with the local network interface 122.

The local memory 124 is shown as containing local machine-executable instructions 130. The local machine-executable instructions 130 contain instructions which enable the local computational system 118 to perform various tasks such as controlling the medical imaging system 108 as well as performing numerical and image processing tasks. The local memory 124 is further shown as containing optional medical imaging system control commands 132. These for example may be control commands for a CT system or pulse sequence commands for controlling a magnetic resonance imaging system. The local memory 124 is further shown as containing measurement data 134.

The measurement data 134 was acquired from the imaging zone 122 and is descriptive of the subject's 120 anatomy within the imaging zone 112. The local memory 124 is further shown as containing a data compression module 136. In this example the data compression module is formed from an encoder portion of an autoencoder. The memory 124 is further shown as containing compressed measurement data 138 that was made by inputting the measurement data 134 into the data compression module 136. In some examples the compressed measurement data 138 is then sent via the network connection 106 to the remote computational system 150 so that it may decompress and then reconstruct a medical image 162.

The local memory 124 is further shown as containing an optional data decompression module 140. The data decompression module 140 is used to decompress the compressed measurement data 138 into optional approximated measurement data 142. The data decompression module 140 is formed from the decoder portion of the autoencoder. There may be a difference between the approximated measurement data 142 and the measurement data 134. The local memory 124 is further shown as containing an optional error of the approximated measurement data 144. This is essentially a difference between the approximated measurement data 142 and the measurement data 134. The memory 124 is then further shown as containing a secondary compression module 146 that is used to compress the error of the approximated measurement data 144 into an optional compressed error data 148. In some examples the compressed error data 148 is transmitted with the compressed measurement data 138. When the compressed measurement data 138 is sent alone, then the compression is lossy. When the compressed error data 148 is sent with the compressed measurement data 138 then this may be used to provide for a lossless or non-lossy decompression scheme.

The receiver portion 104 is now described in detail. As was mentioned above, the receiver portion 104 comprises a remote computer system 116 that comprises a remote computational system 150. The remote computational system 150 is also intended to represent one or more computational systems or computational or computing cores. The remote computational system 150 is shown as being in communication with a remote network interface 152 and a remote memory 154. The remote network interface 152 is used to form the receiving portion of the network connection 106. The remote memory 154 is intended to represent the various types of memories which may be accessible to the remote computational system 150. In one example, the remote memory 154 is a non-transitory storage medium.

The remote memory 154 is shown as containing remote machine-executable instructions 160. The remote machine-executable instructions 160 enable the remote computational system 150 to perform computational and data analysis tasks. The remote machine-executable instructions 160 may also provide instructions for reconstruction of a medical image 162 from at least the approximated measurement data 142. The remote memory 154 is further shown as containing the compressed measurement data 138 that was received via the network interface. The remote memory 154 is further shown as containing the data decompression module 140 that was formed from the decoder portion of the autoencoder. The remote memory 154 is further shown as containing the approximated measurement data 142 that was received by inputting the compressed measurement data 138 into the data decompression module 140. In some instances, the medical image 162 may be reconstructed from the approximated measurement data 142.

The remote memory 154 is further shown as containing an optional compressed error data 148 that is received via the network interface 106. The memory 154 is further shown as containing an optional secondary decompression module 164 that is then used to decompress the optional compressed error data 148 into optional error of the approximated measurement data 144. The error of the approximated measurement data 144 may be used to correct the approximated measurement data 142 and recover the original measurement data 134 which is shown as being optionally stored in the remote memory 154. If the measurement data 134 is recovered in this manner, then the medical image 162 may be reconstructed from the original measurement data 134 instead of the approximated measurement data 142.

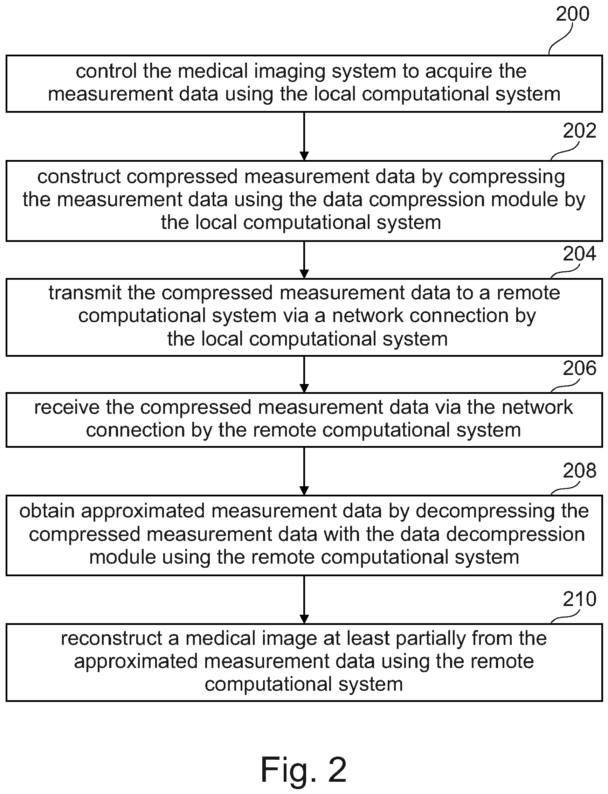

Fig. 2 shows a flowchart which illustrates a method of operating the medical system 100 of Fig. 1. First, in step 200, the medical imaging system 108 is controlled to acquire the measurement data 134. Next, in step 202, the compressed measurement data 138 is constructed by compressing the measurement data 134 using the data compression module 136. Then, in step 204, the compressed measurement data 138 is transmitted to the remote computational system 150 via the network connection 106. Steps 200, 202, and 204 are performed by the transmitter portion 102. The following steps 206, 208, and 210 are performed by the receiver portion 104. After step 204 has been performed step 206 is performed. In step 206 the compressed measurement data 138 is received via the network connection 106. Then, in step 208, the approximated measurement data 142 is obtained by decompressing the compressed measurement data 138 with the data decompression module 140. Finally, in step 210, the medical image 162 is reconstructed from the approximated measurement data 142.

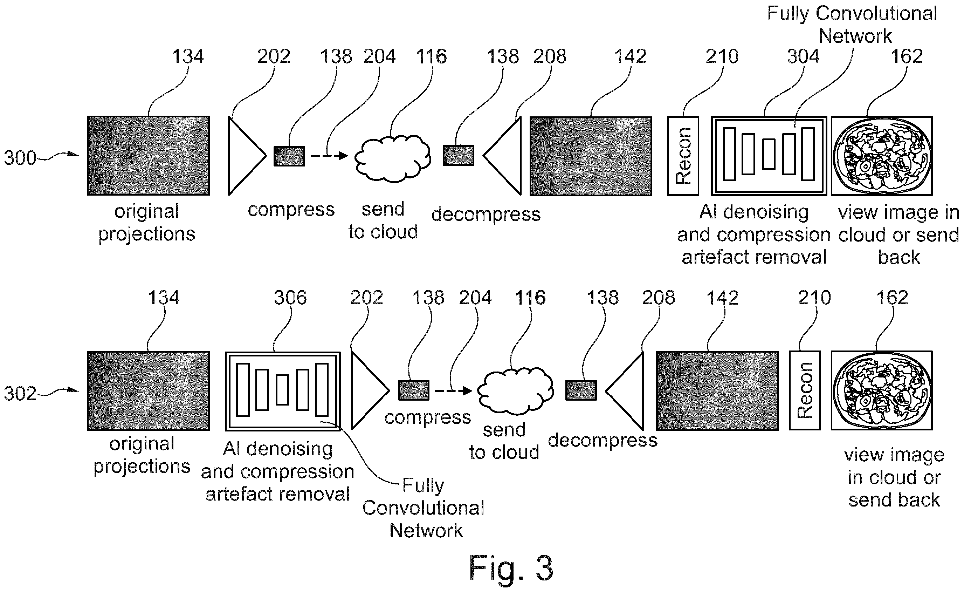

Fig. 3 illustrates a first implementation 300 and a second implementation 302 of methods equivalent to the method illustrated in Fig. 2. The first implementation 300 is described first. First, the measurement data 134 is received. In this case the measurement data are CT projections. Step 202 is then performed where the measurement data 134 is compressed into the compressed measurement data 138. This is then transmitted in step 204 to the remote computer system 116. The compressed measurement data 138 is then decompressed in step 208 to provide the approximated measurement data 142. This is then reconstructed in step 210 to provide the medical image 162. However, as part of the reconstruction 210 or afterwards, there is a noise filtering step 304 that is performed.

The second implementation 302 is very similar to the first implementation except that instead of performing the noise filtering 304 during the reconstruction 210 or immediately afterwards, the measurement data 134 is first put through a noise reduction step before compression 202.

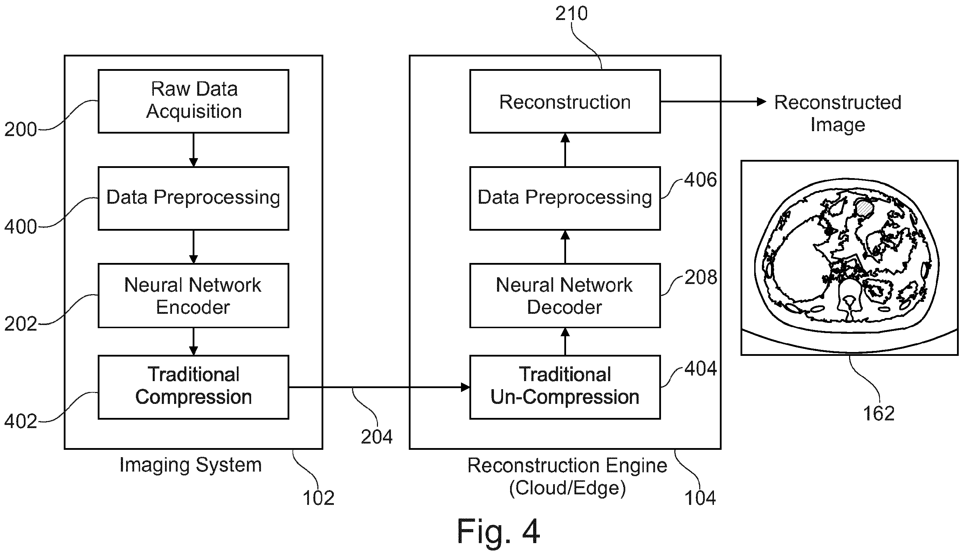

Fig. 4 illustrates a further implementation. In this graphical representation the imaging system represents the transmitter portion 102 and the reconstruction or cloud or edge-based engine represents the receiver portion 104. In the imaging system 102 there is first a raw data acquisition that is equivalent to step 200. Next data preprocessing 400 is performed. This may be equivalent to the noise reduction step 306 in some examples. After this, decoding of the data by a neural network is performed. This is equivalent to step 202. And finally, within the imaging system 102, traditional compression 402 is performed. This means that the compressed measurement data 138 is then compressed with a further traditional or algorithmic compression algorithm.

After this traditional compression 402 the data is then transmitted in step 204 to the reconstruction engine 104. After it is received a traditional un-compression or algorithmic decompression is performed in step 404. This results in providing the approximated measurement data 142 which is then decoded in step 208. This is labeled a neural network decoder. After this is performed there is another data preprocessing step 406 which is performed. In some examples this may be equivalent to step 304 illustrated in Fig. 3. Finally, after this, the reconstruction 210 is performed to provide the medical image 162.

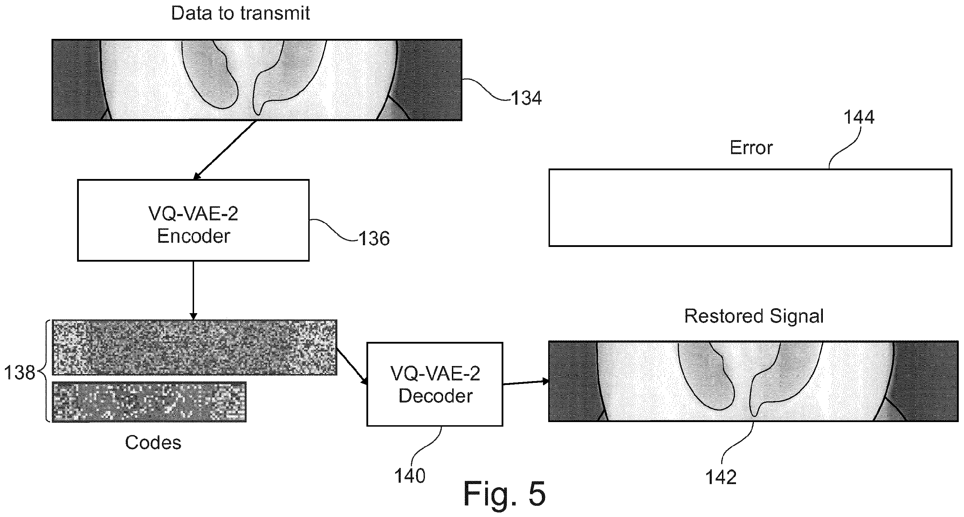

Fig. 5 illustrates the compression and decompression of the measurement data 134 into the approximated measurement data 142. In this example the measurement data 134 is compressed using a VQ-VAE-2 encoder, which functions as the data compression module 136. This then provides two codes or outputs that represent the compressed measurement data 138. These two codes 138 are then input into a VQ-VAE-2 decoder, which in this example is the data decompression module 140, to provide the optional approximated measurement data 142. The box 144 shows the error between the approximated measurement data 142 and the measurement data 134. It should be noted that if the error of the approximated measurement data 144 is also transmitted, the original measurement data 134 may be reconstructed.

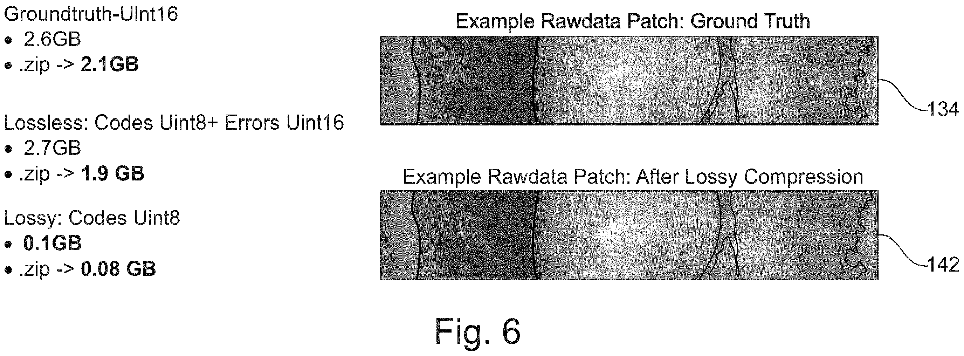

Fig. 6 illustrates the results of the above-described compression technique. The ground truth data, which is equivalent to the measurement data 134, is shown in comparison to data after lossy compression, which is equivalent to the approximated measurement data 142. It can be seen that the ground truth data 134, when compressed using a zip algorithm is reduced from 2.6 gigabytes to 2.1 gigabytes. If a lossless method is used by sending the codes 138, as illustrated in Fig. 5, and the error 144 in a zip file, then the size is reduced from 2.7 gigabytes to 1.9 gigabytes. This represents a small reduction in the size of the compressed data. If a lossy reconstruction is instead used, where only the codes 138, as illustrated in Fig. 5, are transmitted then this lossy compression results in a file size of 0.1 gigabytes. If this is then included into a zip file this can be reduced to 0.08 gigabytes. It can be seen that the lossy compression provided by the encoder 136 and decoder 140 of Fig. 5 reduce results in an enormous reduction in the file size. This for example may reduce the amount of time and resources needed to transmit data for reconstruction into medical images at a remote site.

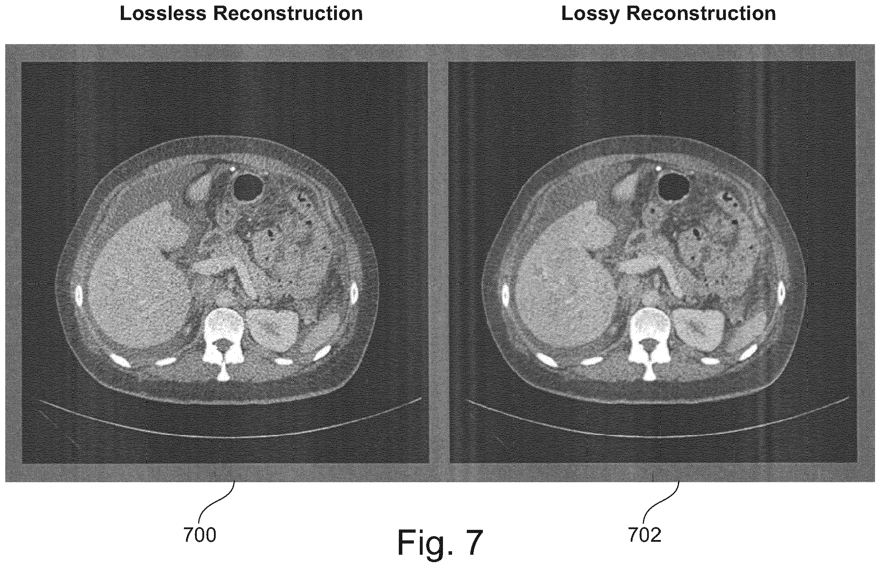

Fig. 7 shows two images. One image is a lossless reconstruction 700 and image 702 is a lossy reconstruction 702. The lossless reconstruction uses both the error 144 and a restored signal 142 to provide the original measurement data 134. The lossy reconstruction only uses the approximated measurement data 142 for the reconstruction. This image illustrates that the lossy reconstruction 702 does an excellent job of reproducing the medical image, although its compression size is over a magnitude of order smaller than using a conventional zip algorithm to compress the data. Another thing which is noticed on examining image 702 is that it has an impaired reduction in noise. This shows that the benefit of reconstructing from the approximated measurement data 142 is not only a reduced transmission time via network connection but also may, in some instances, result in a better medical image 702.

In a further example, that is not illustrated implemented here, one can create tailored training for lossless compression by directly training on an entropy measure, that reflects how well the data can be compressed in the end. In particular we can use the Renyi entropy as a loss function: For example, the Renyi entropy with α = 2 combined with a Parzen estimate of the of the continuous probability density function of Codes and Errors can be used as a differentiable cost function for optimizing directly the compression ratio. The network can then learn a suitable tradeoff between data rates in either the code or the residual errors, which both must be transmitted.

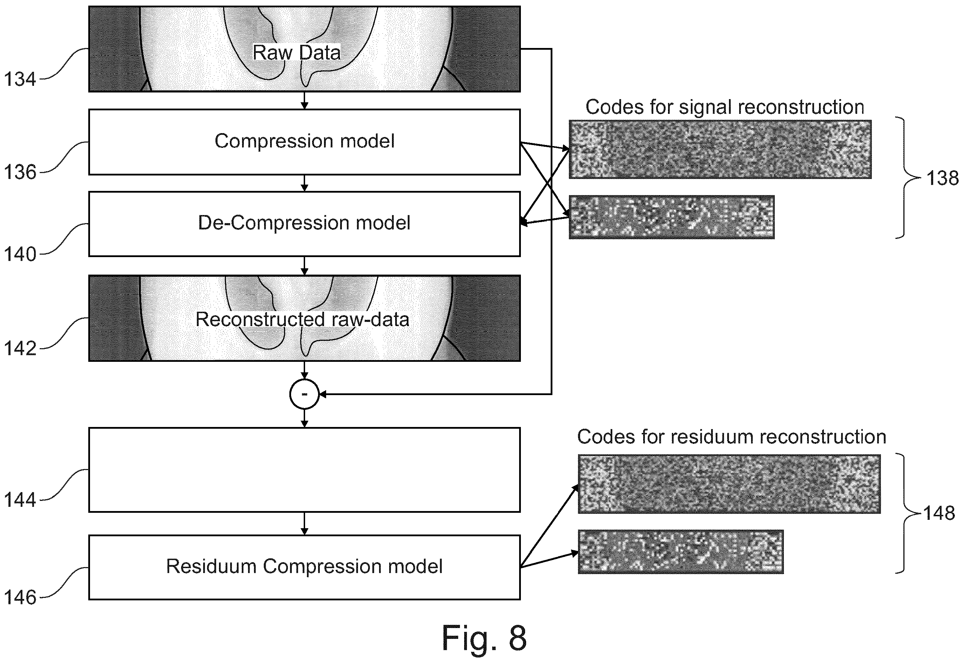

Fig. 8 illustrates a method of using a cascade of compression modules to construct the compressed measurement data and the compressed error data 148. In this method, first the measurement data 134 is received and then it is put into the decompression module 136 which outputs the compressed measurement data 138. The compressed measurement data 138 is then put into the data decompression module 140 to provide the approximated measurement data 142. The measurement data 134 and the approximated measurement data 142 are then subtracted from each other to obtain the error of the approximated measurement data 144. The error of the approximated measurement data 144 is then input into the secondary compression module 146 to provide the compressed error data 148. The compressed measurement data 138 and the compressed error data 148 may then be transmitted from the transmitter portion 102 to the receiver portion 104 for lossless reconstruction.

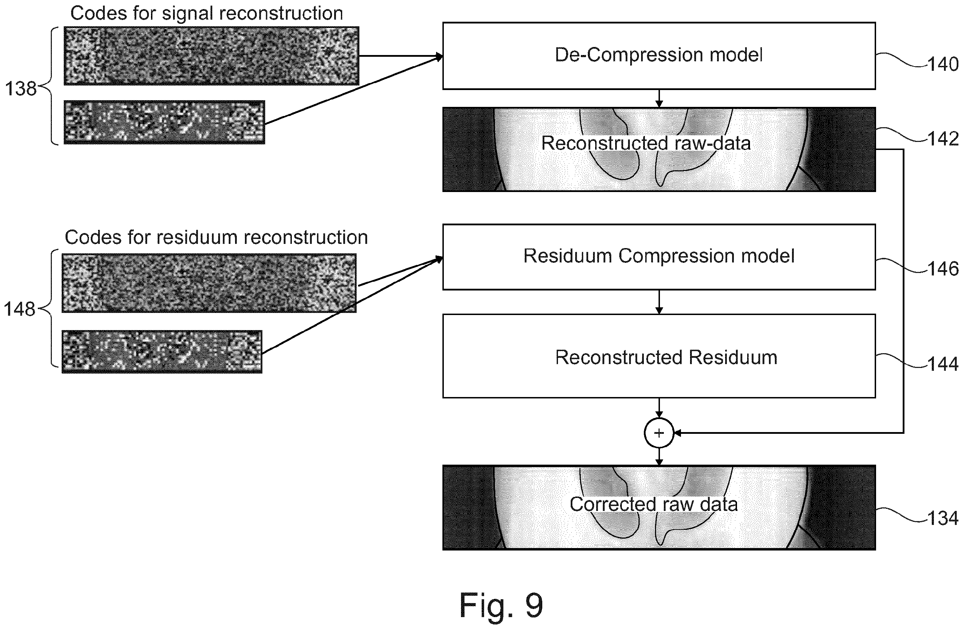

Fig. 9 illustrates the lossless reconstruction of the measurement data 134 after receiving the compressed measurement data 138 and the compressed error data 148. The compressed measurement data 138 is input into the data decompression module 140 to reconstitute the approximated measurement data 142. The compressed error data 148 is then input into the secondary compression module 146 to provide the error of the approximated measurement data 144. The approximated measurement data 142 is then added to the error of the approximated measurement data 144 to recover the original measurement data 134.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Although the invention has been described in reference to specific embodiments, it should be understood that the invention is not limited to these examples only and that many variations of these embodiments may be readily envisioned by the skilled person after having read the present disclosure. The invention may thus further be described without limitation and by way of example only by the following embodiments. The following embodiments may contain preferred embodiments. Accordingly, the term "clause" as used therein may refer to such a "preferred embodiment".

Clause 1. A medical system (100) comprising:
- a local memory (124) storing local machine executable instructions (130) and a data compression module (140), wherein the data compression module comprises an encoder portion of an autoencoder;
- a local computational system (118), wherein execution of the local machine executable instructions causes the local computational system to:
- receive measurement data (134) descriptive of a subject (110);
- construct (202) compressed measurement data (138) by compressing the measurement data using the data compression module, wherein the compressed data is derived from a latent space vector of the autoencoder; and
- transmit (204) the compressed data to a remote computational system (150) via a network connection (106).

Clause 2. The medical system of clause 1, wherein the local memory further stores a data decompression module (136), wherein the data decompression module comprises a decoder portion of the autoencoder.

Clause 3. The medical system of clause 2, wherein the medical system further comprises:
- a remote memory (154) storing remote machine executable instructions (160) and the data decompression module;
- a remote computational system (150), wherein execution of the remote machine executable instructions causes the remote computational system to:
   - receive (206) the compressed measurement data via the network connection;
   - obtain (208) approximated measurement data (142) by decompressing the compressed measurement data with the decompression module; and
   - reconstruct (210) a medical image (162) at least partially from the approximated measurement data.

Clause 4. The medical system of clause 3, wherein execution of the remote machine executable instructions further causes the remote computational system to perform a noise filtering (306) on the approximated measurement data before reconstruction into the medical image.

Clause 5. The medical system of any one of clauses 2, 3 or 4, wherein the local memory further comprises a secondary compression module (146), wherein execution of the local machine executable instructions further causes the local computational system to:
- obtain approximated measurement data (142) by decompressing the compressed measurement data with the decompression module;
- determine an error (144) of the approximated measurement data by calculating a difference between the measurement data and the approximated measurement data;
- obtain compressed error data (148) by compressing the error of the approximated measurement data with the secondary compression module; and
- transmit the compressed measurement data with the compressed data to the remote computational system via the network connection.

Clause 6. The medical system of clause 5, wherein the secondary compression module is implemented as an algorithmic compression module or comprises an encoder portion of a second autoencoder.

Clause 7. The medical system of any one of the preceding clauses, wherein the latent space vector is quantized and/or wherein compression by the data compression module is lossy.

Clause 8. The medical system of any one of the preceding clauses, wherein the autoencoder is any one of the following: a U-Net, a 2-scale Vector-Quantized Variational Autoencoder, and a variational autoencoder.

Clause 9. The medical system of any one of the preceding clauses wherein execution of the local machine executable instructions further causes the local computational system to perform a noise reduction (304) on the measurement data before compression into the compressed data.

Clause 10. The medical system of any one of the preceding clauses, wherein the medical imaging system is any one of the following: a computed tomography system, a magnetic resonance imaging system, a diagnostic ultrasound system, a positron emission tomography system, and a single photon emission tomography system.

Clause 11. A medical system comprising:
- a remote memory (154) storing remote machine executable instructions (160) and a data decompression module (136), wherein the data decompression module comprises a decoder portion of the autoencoder;
- a remote computational system (150), wherein execution of the remote machine executable instructions causes the remote computational system to:
   - receive (206) compressed measurement data (138) via a network connection (106), wherein the compressed measurement data is derived from a latent space vector of the autoencoder; and
   - obtain (208) approximated measurement data (142) by decompressing the compressed measurement data with the decompression module; and
   - reconstruct (210) a medical image (162) at least partially from the approximated measurement data

Clause 12. The medical system of clause 11, wherein the remote memory further comprises a secondary decompression module, wherein execution of the remote machine executable instructions further causes the remote computational system to:
- receive compressed error data (148);
- obtain an error (144) of the approximated measurement data by decompressing the compressed error data with the secondary decompression module; and
- calculate measurement data (134) by correcting the approximated measurement data with the approximated measurement data, and wherein the medical image is reconstructed from the measurement data.

Clause 13. The medical system of any one of the preceding clauses, wherein the medical system further comprises a medical imaging system (108) configured to acquire the measurement data (134) descriptive of the subject (110), and wherein execution of the local machine executable instructions causes the local computational system to control (200) the medical imaging system to acquire the measurement data.

Clause 14. A computer program comprising local machine executable (130) instructions for execution by a local computational system (118) configured for controlling a medical system (100) and a data compression module (140), wherein the data compression module comprises an encoder portion of an autoencoder;
wherein execution of the local machine executable instructions causes the local computational system to:
- receive measurement data (134) descriptive of a subject (110);
- construct (202) compressed measurement data (138) by compressing the measurement data using the data compression module, wherein the compressed data is derived from a latent space vector of the autoencoder; and
- transmit (204) the compressed data to a remote computational system (150) via a network connection (106).

Clause 15. A method of operating a medical system (100),
wherein the method comprises:
- receiving measurement data (134) descriptive of a subject (110);
- constructing (202) compressed measurement data (138) by compressing the measurement data using a data compression module (140) using the local computational system, wherein the data compression module comprises an encoder portion of an autoencoder, wherein the compressed data is derived from a latent space vector of the autoencoder; and
- transmitting (204) the compressed data to a remote computational system (150) via a network connection by the local computational system.

Clause 16. The method of clause 15, wherein wherein the method further comprises:
- receiving (206) the compressed measurement data via the network connection by the remote computational system;
- obtaining (208) approximated measurement data (142) by decompressing the compressed measurement data with a decompression module (136) using the remote computational system; and
- reconstructing (210) a medical image (162) at least partially from the approximated measurement data using the remote computational system.

### REFERENCE SIGNS LIST

- 100: medical system
- 102: transmitter portion
- 104: receiver portion
- 106: network connection
- 108: medical imaging system
- 110: subject
- 112: imaging zone
- 114: local computer system
- 116: remote computer system
- 118: local computational system
- 120: local hardware interface
- 122: local network interface
- 124: local memory
- 130: local machine executable instructions
- 132: medical imaging system control commands
- 134: measurement data
- 136: data compression module
- 138: compressed measurement data
- 140: data decompression module
- 142: approximated measurement data
- 144: error of the approximated measurement data
- 146: secondary compression module
- 148: compressed error data
- 150: remote computational system
- 152: remote network interface
- 154: remote memory
- 160: remote machine executable instructions
- 138: compressed measurement data
- 140: data decompression module
- 142: approximated measurement data
- 162: medical image
- 144: error of the approximated measurement data
- 148: compressed error data
- 164: secondary decompression module
- 200: control the medical imaging system to acquire the measurement data
- 202: construct compressed measurement data by compressing the measurement data using the data compression module
- 204: transmit the compressed data to a remote computational system via a network connection
- 206: receive the compressed measurement data via the network connection
- 208: obtain approximated measurement data by decompressing the compressed measurement data with the decompression module
- 210: reconstruct a medical image at least partially from the approximated measurement data
- 300: first implementation
- 302: second implementation
- 304: noise filtering
- 306: noise reduction
- 400: data processing
- 402: traditional compression
- 404: traditional un-compression
- 406: data preprocessing
- 700: lossless reconstruction
- 702: lossy reconstruction

## Claims

1. A medical system (100) comprising:
- a local memory (124) storing local machine executable instructions (130) and a data compression module (140), wherein the data compression module comprises an encoder portion of an autoencoder;
- a medical imaging system (108) configured to acquire measurement data (134) descriptive of a subject (110);
- a local computational system (118), wherein execution of the local machine executable instructions causes the local computational system to:
- control (200) the medical imaging system to acquire the measurement data;
- construct (202) compressed measurement data (138) by compressing the measurement data using the data compression module, wherein the compressed data is derived from a latent space vector of the autoencoder; and
- transmit (204) the compressed data to a remote computational system (150) via a network connection (106).

2. The medical system of claim 1, wherein the local memory further stores a data decompression module (136), wherein the data decompression module comprises a decoder portion of the autoencoder.

3. The medical system of claim 2, wherein the medical system further comprises:
- a remote memory (154) storing remote machine executable instructions (160) and the data decompression module;
- a remote computational system (150), wherein execution of the remote machine executable instructions causes the remote computational system to:
- receive (206) the compressed measurement data via the network connection;
- obtain (208) approximated measurement data (142) by decompressing the compressed measurement data with the decompression module; and
- reconstruct (210) a medical image (162) at least partially from the approximated measurement data.

4. The medical system of claim 3, wherein execution of the remote machine executable instructions further causes the remote computational system to perform a noise filtering (306) on the approximated measurement data before reconstruction into the medical image.

5. The medical system of any one of claims 2, 3 or 4, wherein the local memory further comprises a secondary compression module (146), wherein execution of the local machine executable instructions further causes the local computational system to:
- obtain approximated measurement data (142) by decompressing the compressed measurement data with the decompression module;
- determine an error (144) of the approximated measurement data by calculating a difference between the measurement data and the approximated measurement data;
- obtain compressed error data (148) by compressing the error of the approximated measurement data with the secondary compression module; and
- transmit the compressed measurement data with the compressed data to the remote computational system via the network connection.

6. The medical system of claim 5, wherein the secondary compression module is implemented as an algorithmic compression module or comprises an encoder portion of a second autoencoder.

7. The medical system of any one of the preceding claims, wherein the latent space vector is quantized and/or wherein compression by the data compression module is lossy.

8. The medical system of any one of the preceding claims, wherein the autoencoder is any one of the following: a U-Net, a 2-scale Vector-Quantized Variational Autoencoder, and a variational autoencoder.

9. The medical system of any one of the preceding claims wherein execution of the local machine executable instructions further causes the local computational system to perform a noise reduction (304) on the measurement data before compression into the compressed data.

10. The medical system of any one of the preceding claims, wherein the medical imaging system is any one of the following: a computed tomography system, a magnetic resonance imaging system, a diagnostic ultrasound system, a positron emission tomography system, and a single photon emission tomography system.

11. A medical system comprising:
- a remote memory (154) storing remote machine executable instructions (160) and a data decompression module (136), wherein the data decompression module comprises a decoder portion of the autoencoder;
- a remote computational system (150), wherein execution of the remote machine executable instructions causes the remote computational system to:
- receive (206) compressed measurement data (138) via a network connection (106), wherein the compressed measurement data is derived from a latent space vector of the autoencoder; and
- obtain (208) approximated measurement data (142) by decompressing the compressed measurement data with the decompression module; and
- reconstruct (210) a medical image (162) at least partially from the approximated measurement data

12. The medical system of claim 11, wherein the remote memory further comprises a secondary decompression module, wherein execution of the remote machine executable instructions further causes the remote computational system to:
- receive compressed error data (148);
- obtain an error (144) of the approximated measurement data by decompressing the compressed error data with the secondary decompression module; and
- calculate measurement data (134) by correcting the approximated measurement data with the approximated measurement data, and wherein the medical image is reconstructed from the measurement data.

13. A computer program comprising local machine executable (130) instructions for execution by a local computational system (118) configured for controlling a medical system (100) and a data compression module (140), wherein the data compression module comprises an encoder portion of an autoencoder, wherein the medical system comprises a medical imaging system (118) configured to acquire measurement data descriptive of a subject (110); wherein execution of the local machine executable instructions causes the local computational system to:
- control (200) the medical imaging system to acquire the measurement data;
- construct (202) compressed measurement data (138) by compressing the measurement data using the data compression module, wherein the compressed data is derived from a latent space vector of the autoencoder; and
- transmit (204) the compressed data to a remote computational system (150) via a network connection (106).

14. A method of operating a medical system (100), wherein the medical system comprises a medical imaging system (108) configured to acquire measurement data (134) descriptive of a subject (110),
wherein the method comprises:
- controlling (200) the medical imaging system to acquire the measurement data using a local computational system (118);
- constructing (202) compressed measurement data (138) by compressing the measurement data using a data compression module (140) using the local computational system, wherein the data compression module comprises an encoder portion of an autoencoder, wherein the compressed data is derived from a latent space vector of the autoencoder; and
- transmitting (204) the compressed data to a remote computational system (150) via a network connection by the local computational system.

15. The method of claim 14, wherein
wherein the method further comprises:
- receiving (206) the compressed measurement data via the network connection by the remote computational system;
- obtaining (208) approximated measurement data (142) by decompressing the compressed measurement data with a decompression module (136) using the remote computational system; and
- reconstructing (210) a medical image (162) at least partially from the approximated measurement data using the remote computational system.
